Europäisches Patentamt

European Patent Office

Office européen des brevets

(11) Publication number: **0 057 450**
**B1**

# (12) EUROPEAN PATENT SPECIFICATION

(45) Date of publication of patent specification: **18.06.86**

(51) Int. Cl.⁴: **A 61 N 1/05**

(21) Application number: **82100643.4**

(22) Date of filing: **01.02.82**

(54) Body implantable lead.

(30) Priority: **02.02.81 US 230694**

(43) Date of publication of application:
**11.08.82 Bulletin 82/32**

(45) Publication of the grant of the patent:
**18.06.86 Bulletin 86/25**

(84) Designated Contracting States:
**DE FR GB NL**

(56) References cited:
**DE-A-3 006 472**
**US-A-4 230 604**

(73) Proprietor: **MEDTRONIC, INC.**
**3055 Old Highway Eight**
**Minneapolis Minnesota 55440 (US)**

(72) Inventor: **Cahalan, Patrick T.**
**11833 Jersey**
**Champlin, MN 55316 (US)**
Inventor: **Jevne, Allan H.**
**1314 153rd Lane N.E.**
**Anoka, MN 55303 (US)**
Inventor: **Coury, Arthur J.**
**2225 Hillside Ave.**
**St.Paul, MN 55108 (US)**
Inventor: **Kallok, Michael J.**
**2097 Violet Lane**
**New Brighton, MN 55112 (US)**
Inventor: **Juncker, David F.**
**16 E.Minnehaha Parkway**
**Minneapolis, MN 55427 (US)**

(74) Representative: **Schwan, Gerhard, Dipl.-Ing.**
**Elfenstrasse 32**
**D-8000 München 83 (DE)**

EP 0 057 450 B1

Courier Press, Leamington Spa, England.

## Description

Background of Prior Art

Electrical stimulation of the body for medical purposes is well known in the prior art. An example of a device for this purpose is the well-known cardiac pacemaker. In the pacemaker context, as well as other body stimulation contexts, the stimulation is delivered to the desired body site by an electrode component of a lead.

A variety of body implantable electrical leads for the delivery of electrical stimulation energy to a desired body site are known in the prior art. Recent years have seen increased usage of transvenous leads which can deliver the stimulation energy to the desired body site while significantly lowering patient risk and morbidity. For example, the use of a transvenous/endocardial lead eliminates the necessity of a thoracotomy while delivering stimulation energy to the heart to provide a pacemaking function.

Such implantable leads may be used for sensing as well as stimulating.

In many stimulation and sensing contexts, maintenance of a reliable electrical contact at the desired electrode site has proven difficult. Interactions between the lead and the body at the contact site can vitiate the desired effects of the stimulation. For example, material reactions may encourage fibrosis. In the pacemaking context, fibrosis is believed to be a major factor in the increase in chronic threshold that is usually experienced. Also, mechanical trauma may result in inflammation of the tissue to be stimulated. Such inflammation may alter the response of the tissue to the stimulation energy, both acutely and chronically.

In chronic use of such leads, it has been observed that the contractions of the heart against the lead and the mechanical pressure applied against the heart by the lead may traumatize the contacted tissue. The traumatized tissue may form scar tissue around the electrode(s) to reduce the stress placed upon the endocardium below the tissue damage limit. In chronic experience, it is observed that the threshold currents sufficient to stimulate the heart increase as the scar tissue is formed until the thickness of the scar tissue stabilizes. The required energy to stimulate the heart is thereby increased because of the additional potential drop across the nonresponsive cardiac cells. A discussion of this mechanism in conjunction with the realization of optimum electrode surface area appears in the paper entitled "Comparison of Power Sources for Advanced Pacemaker Applications" by Rasor, Spickler and Clabaugh, *Proceedings of the Seventh Intersociety Energy Conversion Engineering Conference*, January 1972, pages 752—760.

Other interactions, such as electrochemical and polarization effects, between the lead and body tissue at the contact site, can result in the occurrence of undesirable events. In some circumstances where electrical stimulation of body tissue is indicated, the tissue portion to be stimulated is irritable. The placement of a pacemaking lead in such a case may induce a cardiac arrhythmia. The presence of the lead may also promote thrombus formation.

Pressure-induced trauma are thought to be the leading cause of enlarged fibrotic encapsulation of the electrode tip.

Cardiac tissue is also occluded under the tip and deprived of access to oxygen and other nutrients carried by the blood while also being deprived of the ability to transfer waste products from the tissue into the blood. A large number of tissue cells are so occluded by existing endocardial electrode tips. Cells of heart tissue must communicate with the blood in the ventricles of the heart by way of diffusion due to the lack of capillaries near the endocardial surface.

The interactions noted above have long been recognized and efforts have been made to ameliorate their consequences. For example, leads have been configured to reduce mechanical trauma and the response of irritable tissue during lead placement. Materials have been selected for the lead body and electrodes to minimize fibrosis. Thrombus formation may also be countered by the administration of suitable drugs.

The administration of drugs to counter the undesirable interactions between the lead and body noted above has not gained widespread acceptance in that it has heretofore required a systemic treatment to counter a localized interaction. Also, lead configuration must take into account other factors such as the efficiency of the delivery of the stimulation energy, the ease of lead placement, maintenance of the desired electrode position and reliability of the lead over extended periods of time. An accommodation of these interests has resulted in leads whose configuration necessarily results in undesirable interactions between the lead and body.

One way to avoid the waste of energy due to polarization effects has been described by Parsonnet et al., "Clinical Use Of A New Transvenous Electrode", *Ann. N.Y. Acad. Sci.*, Vol. 167: 756, 1969. This article describes what is termed a "Differential Current Density Electrode". In this system there is a low current density at the metal electrode to avoid polarization and a high current density at the contact to tissue to produce a significant field strength to activate responsible cells. As a result, stimulation thresholds were found which were lower than that of conventional metal electrodes.

DE—A—3 006 472 describes a body implantable lead for electrically stimulating body tissue, including a lead body comprising a sheath of insulating material, a metallic wire extending through the sheath, a metallic tip located at the end of the wire and covered by a non-metallic coating composed of a compound of the metal in the tip, and an ion-conducting polymer membrane which covers the non-metallic coating, has a thickness from 1 to 100 μm and prevents the metallic tip and the non-metallic coating from contacting body tissue thereby preventing an electrochemical reaction between body tissue and the metal tip when the lead is implanted.

Brief Summary of the Invention

A body implantable lead including a lead body comprising a sheath of insulating material; a conductive polymer electrode carried by the lead body for providing electrical contact between the body implantaable lead and living tissue, and metal lead means extending through the sheath and being connected to the conductive polymer electrode within the sheath, in conformity with the present invention is characterized in that the conductive polymer electrode essentially consists of a conductive hydrogel body which is at least thick enough to contain the Helmholtz layer formed around the end surface of the metal lead means.

The conductive polymer which may be cast to shape or formed in situ. The polymer electrode avoids the polarization phenomena associated with metal electrodes in a conductive fluid as is encountered in living bodies. The electrode of the invention provides a transition zone wherein it is believed the conversion of electron flow to ionic flow occurs, i.e., the Helmholtz layer. With metal electrodes, the Helmholtz layer is external to the electrode and is such that cells in its vicinity are disrupted or destroyed. With the polymer electrode of the invention a transitional synthetic layer is provided in the electrode where this reaction can take place.

Significant advantages of the invention are: reduced tissue trauma; use of inexpensive gel electrode rather than expensive precious metal electrodes; improved electrode-tissue interface, mechanically, chemically and electrically; use of non-corroding electrode material, improved sensing due to low polarization, improved capture efficiency, lower thresholds, lower current drain, and protection of the living cells from any electron/ion conversion reaction.

Particularly, the conductive polymer electrode of the present invention defines a body of soft, compliant, water swellable, water insoluble material non-toxic to tissues and fluids. The hydrogel is permeable to water, oxygen, $CO_2$ and the like so as to allow their diffusion through the cushioning body both to and away from the occluded tissue cells at the contact site. It is ionically conductive thereby providing electrical contact between the metal lead means and the tissue.

Brief Description of the Drawings

Figure 1 illustrates a portion of a body implantable lead constructed in accordance with the preferred embodiment of the present invention.

Figure 2 is an end view of the body implantable lead shown in Figure 1.

Figure 3 is a schematic showing of an electrode for a bipolar lead.

Detailed Description of the Invention

A conductive polymer gel is used as the stimulating and sensing medium in an implantable transvenous lead, particularly in a cardiac pacing lead, from considerations of the electrochemistry at the electrode/tissue (electrolyte) interface. The charge carriers in metal are electrons whereas charge is carried by ions in electrolyte solutions. When metal is immersed in an electrolyte, charge flows from one phase to another until the electrochemical potentials of the metal and electrolyte phases are equal. This results in the build-up of a layer of charge, called the Helmholtz layer, around the surface of the electrode. This phenomen is termed polarization and results in a dissipative energy loss that shortens pulse generator battery life and also makes sensing difficult. Electrochemical reactions may also result in corrosion of the electrode metal.

In addition to the detrimental effects of the Helmholtz layer on device operation, there is some question as to whether the chronic presence of a charge layer near the tissue causes myocardial or endocardial pathologic processes. For example, the Helmholtz layer may chronically alter the local pH in the vicinity of the electrode. The chronic imbalance of charge may affect the membrane properties such that changes in myocardial or endocardial cell depolarization threshold occur.

Because of the known, as well as the unknown or speculative, effects attributable to the Helmholtz layer, the gel electrode of the invention is proposed as a solution for, among other things, minimizing these adverse consequences of polarization. Because the metal electrolyte interface in the gel electrode design is removed from the near vicinity of the tissue, the Helmholtz layer is believed to be encapsulated in the gel. Since charge transport is by ionic movement in the gel, the ions can diffuse into and out of the gel freely, presumably a charge layer is not established at the surface of the gel at which tissue contact is established. Improved sensing and lower stimulation thresholds result from the combined effects of elimination of the Helmholtz layer and minimizing charge imbalances in the vicinity of the myocardial cells.

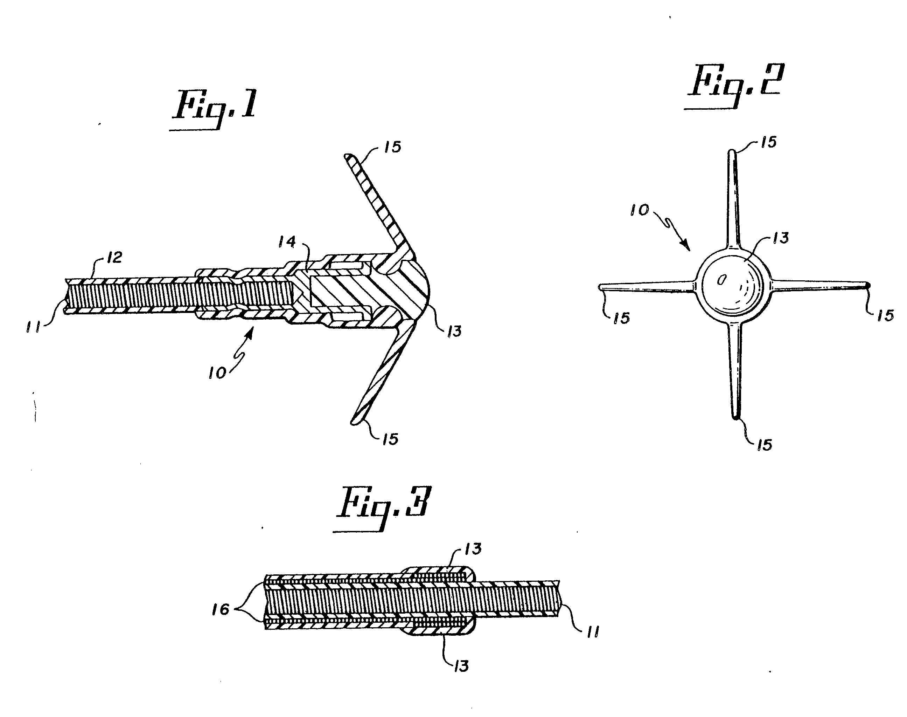

Referring now to Figures 1 and 2, there is illustrated a body implantable lead constructed in accordance with the present invention. The lead body, designated 10, is comprised of a coiled, flexible conductor 11 and an overlying sheath 12. Coiled, flexible conductor 11 may be of any known design and preferably has a central aperture which will accept a stylet (not shown) to provide stiffness during lead placement, in known manner. The lead body may also be non-linear such that stylet insertion will straighten the lead body to facilitate transvenous placement, also in known manner. To facilitate the flexibility requirements of the present invention as well as for reliability considerations, flexible conductor 11 may be a multi-filar member. Conductor 11 is in electrical communication with a polymer gel electrode 13 by means of a metal crimp-sleeve 14 and a source of stimulation energy (not shown) for the delivery of stimulation energy to a desired body site. Sheath 12 may be a preformed tubular member, preferably of a body compatible polyurethane, to overlie conductor 11 and provide electrical insulation therefor, in known manner. Also, as is known, lead body 10 may be tined as indicated at 15.

In the prior art, many lead bodies are formed of a molded silicone. A preferable material for sheath 12 and lead body 10 is a body-compatible polyurethane. Such polyurethanes meet the flexibility requirements of transvenous leads and are typically less thrombogenic than silicone and more resistant to tearing and cutting. In general, the physical characteristics of polyurethane are more suited to transvenous leads than those of silicone, although silicone or any other body compatible material may be employed in practicing the present invention.

Polymer gel electrode 13 may be provided in various configurations. It may be pre-cast or polymerized in situ, as desired.

In the embodiment shown, lead body 10 defines a cavity which contains polymer electrode 13 with a rounded end portion thereof extending through the open distal end of the lead body. The rounded end provides electrical contact between living tissue and lead means 11.

A wide variety of lead configurations, including bipolar as well as unipolar, and electrode body configurations consistent with the tissue contacting requirements of the lead may be utilized. A ring-electrode for a bi-polar lead is illustrated in Figure 3 which includes a second lead means 16 and a band 13 of polymer gel forming an electrode.

Electrode body 13 consists essentially of a conductive hydrogel thick enough to accommodate the Helmholtz layer. Typically, thicknesses i.e., the dimension of body 13 extending out of the lead body, will range from about 0.5 mm to about 1.0 mm. Such electrode body has been found to provide low mechanical trauma and high electrochemical interface with endocardial tissue.

Hydrogels used for the lead of the invention are ionically conductive, soft, compliant, water swellable and inert to body tissues and fluids. Body 13 is also ionically conductive and permeable thereby establishing electrical communication between lead means 11 and the contacted tissue as well as providing a diffusion path to and away from the occluded tissue site.

Generally speaking, a hydrogel having the properties above described will comprise a coherent, three-dimensional polymeric network capable of imbibing water without dissolving. Usually, insolubility in water is provided by incorporating a crosslinked structure in the polymer. Hydrogels or water-containing gels may be comprised of water and various chemical substances including gelatin; polysaccharides; crosslinked acrylamide polymers; hydroxyethylmethacrylate polymers; crosslinked polyhydroxyethylacrylate; polymerized, crosslinked 2-acrylamide-2-methylpropane sulfonic acid or one of its salts such as the sodium or potassium type; crosslinked polyvinylpyrrolidone; polyacrylic acid; copolymers of the aforementioned monomers with each other, and copolymers of the aforementioned monomers with other monomers such as styrene or other non-hydrogel forming monomers.

Hydrogels of low conductivity may be made conductive for purposes of this invention by incorporating salts such as sodium chloride or potassium chloride and like electrolytic salts in the hydrogel. This is easily accomplished by dissolving a salt in a monomer-water-initiator solution and polymerizing. Other methods of preparation may also be used.

The specific preferred hydrogels are crosslinked polyacrylamide and crosslinked polymerized 2-acrylamido-2-methylpropane sulfonic acid or one of its salts. Volume 15, pages 273—291 of the *Encyclopedia of Polymer Science Technology* (1971), John Wiley Publishers, provides a section entitled HYDROGELS which describes the preparation of a variety of water-imbibing polymers. The content thereof is incorporated herein by reference.

Example — Polyacrylamide Hydrogel

The following ingredients were dissolved in 52.5 grams of deionized water: 40.0 grams acrylamide, 2.0 grams sodium chloride, 8.0 cc of a 1% aqueous solution of methylene-bis-acrylamide. Nitrogen was vigorously bubbled through the resultant solution for 2.5 minutes. The following reagents were added to the solution simultaneously with stirring: 0.5 cc of a .38% solution of potassium bisulfite, 0.5 cc of a .38% solution of potassium persulphate, and 0.5 cc of a .24% solution of ferrous sulfate.

A portion of the resultant mixture was withdrawn into a syringe and ejected into the cavity of electrode tip 13 under a nitrogen atmosphere. The mixture was cured to form the hydrogel by allowing it to stand under the nitrogen atmosphere at room temperature until solidified.

Some of the subject matter disclosed herein is claimed in our European patent application EP—A—00 57 451.

**Claims**

1. A body implantable lead including a lead body (10) comprising a sheath (12) of insulating material; a conductive polymer electrode (13) carried by the lead body for promiding electrical contact between the body imptantable lead and living tissue, and metal lead means (11, 16) extending through the sheath and being connected to the conductive polymer electrode within the sheath, characterized in that the conductive polymer electrode essentially consists of a conductive hydrogel body (13) which is at least thick enough to contain the Helmholtz layer formed around the end surface of the metal lead means.

2. The lead of claim 1 wherein the dimensions of the hydrogel body (13) are from 0.5 mm to 1.0 mm.

3. The lead of claim 1 or 2 wherein the polymer electrode (13) is carried at the distal end of the lead body (10).

4. The lead of claim 3 wherein the lead body (10) defines a cavity having an opening at the distal end thereof, and the polymer electrode (13) is contained in the cavity with a portion thereof extending through the cavity opening for providing a lead contact surface for contacting living tissue.

5. The lead of any of the preceding claims wherein the lead is of the transvenous type.

6. The lead of any of the preceding claims wherein the tissue contacting portion of the polymer electrode (13) defines a rounded configuration.

7. The lead of any of the preceding claims wherein the polymer is an acrylamide.

8. The lead of any of the preceding claims wherein the hydrogel includes a methylacrylate polymer.

9. The lead of any of the preceding claims wherein the hydrogel includes a crosslinked polymer.

10. The lead of claim 9 wherein the hydrogel includes a crosslinked ethylacrylate polymer.

11. The lead of claim 9 wherein the hydrogel includes a crosslinked polymer of 2-acrylamido-2-methylpropane sulfonic acid or one of its salts.

12. The lead of claim 9 wherein the hydrogel includes a crosslinked vinylpyrrolidone polymer.

13. The lead of any of the preceding claims wherein the hydrogel includes a copolymer.

14. The lead of claim 13 wherein the copolymer is comprised of at least two copolymerized hydrogel-forming monomers.

15. The lead of claim 13 wherein the copolymer is comprised of at least one hydrogel-forming monomer and one non-hydrogel-forming monomer copolymerized together.

**Patentansprüche**

1. Im Körper implantierbare Leitung mit einem Leitungskörper (10), der eine Ummantelung (12) aus isolierendem Werkstoff, eine von dem Leitungskörper getragene, leitende Polymerelektrode (13) zur Herstellung von elektrischem Kontakt zwischen der im Körper implantierbaren Leitung und lebendem Gewebe, und metallische Leitungsmittel (11, 16) aufweist, welche durch die Ummantelung hindurchreichen und mit der leitenden Polymerelektrode innerhalb der Ummantelung verbunden sind, dadurch gekennzeichnet, daß die leitende Polymerelektrode im wesentlichen aus einem leitenden Hydrogelkörper (13) besteht, der mindestens ausreichend dick ist, um die Helmholtz-Schicht zu enthalten, die um die Endfläche der metallischen Leitungsmittel herum ausgebildet wird.

2. Leitung nach Anspruch 1, wobei die Abmessungen des Hydrogelkörpers (13) zwischen 0,5 mm und 1,0 mm liegen.

3. Leitung nach Anspruch 1 oder 2, wobei die Polymerelektrode (13) an dem distalen Ende des Leitungskörpers (10) sitzt.

4. Leitung nach Anspruch 3, wobei der Leitungskörper (10) einen Hohlraum bildet, der an seinem distalen Ende eine Öffnung aufweist und die Polymerelektrode (13) in dem Hohlraum untergebracht ist, wobei ein Teil der Elektrode durch die Hohlraumöffnung hindurchreicht, um eine Leitungskontaktfläche zum Kontaktieren von lebendem Gewebe zu bilden.

5. Leitung nach einem der vorhergehenden Ansprüche, wobei die Leitung als transvenöse Leitung ausgebildet ist.

6. Leitung nach einem der vorhergehenden Ansprüche, wobei der mit Gewebe in Kontakt kommende Teil der Polymerelektrode (13) eine abgerundete Konfiguration bildet.

7. Leitung nach einem der vorhergehenden Ansprüche, wobei das Polymer ein Acrylamid ist.

8. Leitung nach einem der vorhergehenden Ansprüche, wobei das Hydrogel ein Methylacrylatpolymer aufweist.

9. Leitung nach einem der vorhergehenden Ansprüche, wobei das Hydrogel ein vernetztes Polymer aufweist.

10. Leitung nach Anspruch 9, wobei das Hydrogel ein vernetztes Äthylacrylatpolymer aufweist.

11. Leitung nach Anspruch 9, wobei das Hydrogel ein vernetztes Polymer von 2-Acrylamid-2-methylpropan-Sulfonsäure oder einem ihrer Salze aufweist.

12. Leitung nach Anspruch 9, wobei das Hydrogel ein vernetztes Vinylpyrrolidonpolymer aufweist.

13. Leitung nach einem der vorhergehenden Ansprüche, wobei das Hydrogel ein Copolymer aufweist.

14. Leitung nach Anspruch 13, wobei das Copolymer aus mindestens zwei copolymerisierten, hydrogel-bildenden Monomeren besteht.

15. Leitung nach Anspruch 13, wobei das Copolymer aus mindestens einem hydrogel-bildenden Monomer und einem nichthydrogel-bildenden Monomer besteht, die zusammen copolymerisiert sind.

**Revendications**

1. Conducteur implantable dans le corps comprenant un corps de conducteur (10) constitué par une gaine (12) de matière isolante, une électrode (13) d'un polymère conducteur supportée par le corps de conducteur pour établir un contact électrique entre le conducteur implantable dans le corps et un tissue vivant, et un conducteur métallique (11, 16) passant dans la gaine et connecté à l'électrode d'un polymère conducteur dans la gaine, caractérisé en ce que l'électrode d'un polymère conducteur consiste essentiellement en un corps d'hydrogel conducteur (13) qui est au moins suffisamment épais pour contenir la couche de Helmholtz, formée autour de la surface d'extrémité du conducteur métallique.

2. Conducteur selon la revendication 1, dans lequel les dimensions du corps d'hydrogel (13) sont comprises entre 0,5 mm et 1,0 mm.

3. Conducteur selon la revendication 1 ou 2, dans lequel l'électrode de polymère (13) est supportée à l'extrémité distale du corps de conducteur (10).

4. Conducteur selon la revendication 3, dans lequel le corps du conducteur (10) définit une cavité avec une ouverture à son extrémité distale, et l'électrode de polymère (13) se trouve dans la cavité avec une de ses parties passant par l'ouverture de la cavité pour présenter une surface de contact de conducteur destinée au contact avec un tissu vivant.

5. Conducteur selon l'une quelconque des revendications précédentes, dans lequel le conducteur est du type transveineux.

6. Conducteur selon l'une quelconque des revendications précédentes dans lequel la partie en contact avec un tissu, de l'électrode de polymère (13), a une configuration arrondie.

7. Conducteur selon l'une quelconque des revendications précédentes dans lequel le polymère est un acrylamide.

8. Conducteur selon l'une quelconque des revendications précédentes, dans lequel l'hydrogel contient un polymère de méthylacrylate.

9. Conducteur selon l'une quelconque des revendications précédentes, dans lequel l'hydrogel contient un polymère réticulé.

10. Conducteur selon la revendication 9, dans lequel l'hydrogel contient un polymère d'éthylacrylate réticulé.

11. Conducteur selon la revendication 9, dans lequel l'hydrogel contient un polymère réticulé d'acide sulfonique-2-acrylamido-2-méthylpropane ou l'un de ses sels.

12. Conducteur selon la revendication 9, dans lequel l'hydrogel contient un polymère de vinylepyrolidole réticulé.

13. Conducteur selon l'une quelconque des revendications précédentes, dans lequel l'hydrogel contient un copolymère.

14. Conducteur selon la revendication 13, dans lequel un copolymère est constitué par au moins deux monomères copolymérisés formant l'hydrogel.

15. Conducteur selon la revendication 13, dans lequel le copolymère est constitué par au moins un monomère formant l'hydrogel et un monomère ne formant pas d'hydrogel, copolymérisés ensemble.

_Fig.1_

_Fig.2_

_Fig.3_